Europäisches Patentamt

European Patent Office

Office européen des brevets

(19)

(11) Publication number: **0 056 241**
**B1**

(12) **EUROPEAN PATENT SPECIFICATION**

(45) Date of publication of patent specification: **16.04.86**

(21) Application number: **82100036.1**

(22) Date of filing: **06.01.82**

(51) Int. Cl.⁴: **A 61 B 10/00**

(54) Apparatus for collecting saliva.

(30) Priority: **12.01.81 US 224306**

(43) Date of publication of application:
**21.07.82 Bulletin 82/29**

(45) Publication of the grant of the patent:
**16.04.86 Bulletin 86/16**

(84) Designated Contracting States:
**AT BE CH DE FR GB IT LI LU NL SE**

(56) References cited:
**DE-A-1 937 021**
**US-A-2 905 169**
**US-A-4 014 322**
**US-A-4 184 483**
**US-A-4 232 673**

(73) Proprietor: **MetPath, Inc.**
**One Malcolm Avenue**
**Teterboro New Jersey 07608 (US)**

(72) Inventor: **Brown Paul, Dr.med.**
**174 Elmsley Court**
**Ridgewood New Jersey (US)**
Inventor: **O'Brien, Joseph, Dr.med.**
**302 Manner Road**
**Ridgewood New Jersey (US)**

(74) Representative: **Gille, Christian, Dipl.-Ing.**
**Redies , Redies, Türk & Gille Bruckner Strasse 20**
**D-4000 Düsseldorf 13 (DE)**

Courier Press, Leamington Spa, England.

## Description

The invention relates to an apparatus for collecting from a test subject a volume of saliva as specimen, said apparatus having a vessel for receiving saliva absorptive material and means for expressing the saliva from said saliva absorptive material for collecting it within said vessel.

Standardized specimen receptacles for collecting, storing and transporting body fluids, such as blood or urine, for later use in diagnostic testing are common and well-known. In particular, a device for collecting aseptic liquid specimens comprising a container and a cylindrical sponge element mounted upon a rod in contact with a cylindrical plate in order to compress the sponge for expressing the liquid content of said sponge is known (US—A—4 014 322). The liquid expressed from the sponge drops into the lower portion of the container through openings in a plate at the upper portion of the container onto which the sponge can be compressed. Since the absorptive material is to be placed on a rod it is difficult to collect liquid specimen, especially saliva specimen, so that this known device is not easy to handle for collecting specimens of saliva.

The object of the invention is to provide a new and improved apparatus for collecting saliva which is easily to be handled.

This object is solved by the present invention with an apparatus as specified in claim 1. Suitable improvements are subject matter of the subclaims.

In accordance with the invention saliva specimens are collected by placing an absorbent flavored mass in the mouth of a test subject until a quantity of saliva has been absorbed. The absorbed saliva is then expressed from the mass into the specimen vial of the collecting apparatus by means of the screw jack-piston. A preservative is included within the specimen vial to preserve the saliva specimen and to prevent its contamination before diagnostic testing.

With the apparatus of the present invention saliva samples can effectively and systematically be collected and the specimen collecting apparatus is easy to manipulate and inexpensive to manufacture. The elements of the specimen collecting apparatus may all be made of inexpensive plastic material making the apparatus readily disposable after a single use. Moreover, the plastic construction of the specimen collecting apparatus enables it to be safely and reliably shipped or mailed to laboratories for testing.

The saliva collecting apparatus of the present invention can be used for human testing as well as animal testing.

For a more detailed understanding of the present invention and for greater appreciation of its attending advantages one embodiment of the saliva collecting apparatus of the present invention is described in detail below with reference to the drawings, in which

Fig. 1 is an exploded view of the apparatus embodying the principles of the invention and

Fig. 2 is a vertical section of the apparatus with its parts in the operational position for expressing saliva from a mass of absorbent material.

In accordance with the principles of the invention, an absorbent mass 9 is provided of a size and shape to fit easily within the test subject's mouth for mastication, but not so small as to present a risk of being swallowed. This mass 9 may be made, for example, of cellulose sponge or absorbent thermo-plastic foam, such as a polyester or polyurethane foam.

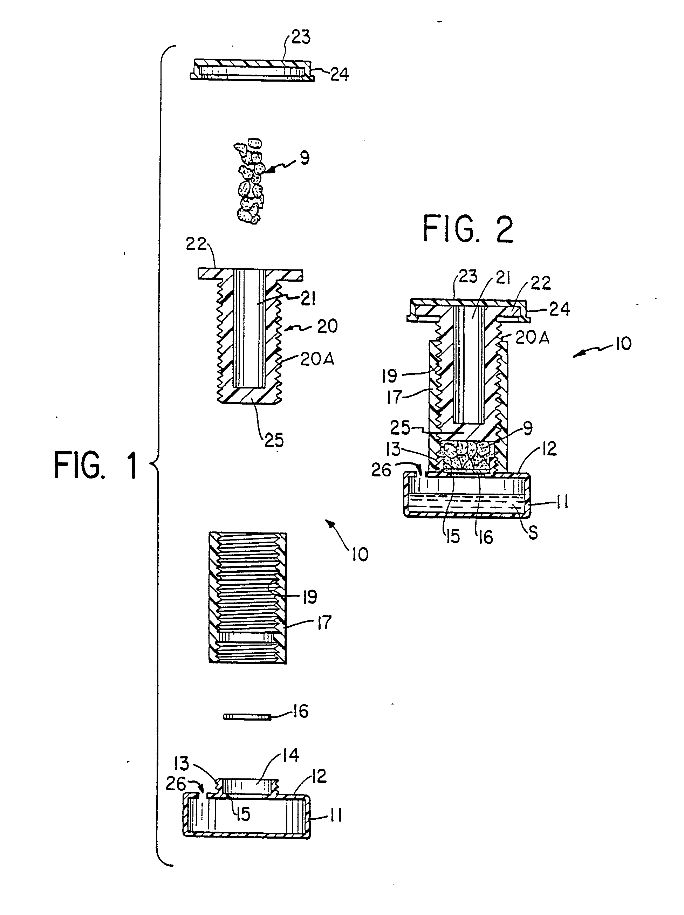

In Fig. 1, the specimen collector 10 of the present invention appears in exploded form. The base is a cylindrical specimen vial 11, in which the saliva specimen will be stored until tested. The vial 11 has a top wall 12 supporting an upwardly projecting cylindrical threaded flange 13 defining an aperture 14. A horizontal projecting flange 15 of the vial 11 supports a removable filter disc 16 which completely covers the aperture 14.

Threaded upon the flange 13 is a hollow, cylindrical barrel 17. The interior of the barrel 17 is of sufficient volume to hold the mass 9 when saturated. Interior coarse threads 19 in the barrel 17 matingly receive a threaded piston 20. Specifically, the piston 20 itself has a hollow interior 21, open at the fingergrip top 22, to serve as a storage space for the mass 9. The piston 20 has a compression head 25 at its bottom surface. A snap-on cover 23, having a resilient downwardly extending flange 24, engages the fingergrip top 22 and sealing the interior 21 from contamination by contact with the outside atmosphere.

In accordance with the invention and to encourage the test subject to salivate while chewing on the mass 9, the mass 9 may be impregnated with a flavor, such as chocolate or cherry, the flavoring agent being inert and digestible. This will also help overcome any reluctance on the part of the test subject to place the mass 9 in his or her mouth.

To obtain the saliva specimen, the procedure of the invention is as follows: The cover 23 is snapped off and the mass 9 is removed from the interior 21. The test subject places it in his or her mouth and masticates it until it has become saturated with saliva. The piston 20 is unscrewed from the extruder barrel 17. The saturated mass is deposited in the extruder barrel 17 and the piston 20 is inserted and threaded down until the saliva specimen has been expressed from the mass 9 by the action of the compression head 25 against the filter disc 16 and has passed into the specimen vial 11. Ambient air within the confines of the barrel will be forced out through the space between threads 19 and the threads 20 on the plunger, as will be understood. When the fit between strands 19, 20a is tight rather than loose, a relief vent 26 is formed in vial 11. The filter disc 16 prevents particles of the mass 9 or other detritus and/or bacteria from being collected, and may be held in place by adhesive at the periphery or by mechanical locking means. The saliva specimen is then stored in the vial 11 until it is used for diagnostic testing. The cap 23 may be sized and configured, if desired, to be used to seal the vial 11 rather than using a separate cap.

To preserve the specimen until testing, the specimen vial may be provided with a preservative. Alternatively, if desired or necessary, a reactant for an immediate processing step may be provided.

## Claims

1. Apparatus for collecting from a test subject a volume of saliva as a specimen, said apparatus having a vessel for receiving saliva absorptive material and means for expressing the saliva from said saliva absorptive material for collecting it within said vessel, characterized in that said vessel comprises a hollow cylindrical barrel (17) having first and second open ends, and a collecting vial (11) for receiving saliva releasably connected to the first end of said barrel, said vial having an aperture (14) at one end and a filter disc (16) covering said aperture which is engaged with the first end of said barrel (17), a piston element (20) movably inserted into the second end of said hollow cylindrical barrel (17) so that it compresses a mass of said saliva-absorptive material (9) received within the cylindrical barrel (17) between said piston element (20) and the first end of said cylindrical barrel (17) covered by said filter disc (16) when moved towards said filter disc (16) to thereby express the saliva content of said mass such that the expressed saliva will flow through the filter disc (16) into said collecting vial (11).

2. Apparatus according to claim 1, characterized in that a storage space (21) is formed in said piston element for selectively, temporarily storing said mass of saliva-absorptive material (9).

3. Apparatus according to claim 2, characterized in that a removable cover element (23) is releasably connected to said piston element (20) for enclosing said storage space (21).

4. Apparatus according to any one of claims 1 to 3, characterized in that said mass (9) of saliva-absorptive material comprises a cellulose sponge.

5. Apparatus according to any one of claims 1 to 4, characterized in that the first screw threads (19) are formed on the internal surface of said hollow cylindrical barrel (17) and that second screw threads (20a) are formed on the external surface of said piston element (20) which is telescopically inserted into the second end of said barrel (17) in a manner such that said second screw threads (20a) engage said first screw threads (19).

## Revendications

1. Appareil pour recueillir sur un sujet à examiner un volume de salive constituant un échantillon, cet appareil comprenant un récipient destiné à recevoir une matière absorbante de la salive, et des moyens servant à exprimer la salive de ladite matière absorbante de la salive pour la recueillir dans ledit récipient, caractérisé en ce que ledit récipient comprend un tube cylindrique creux (17) possédant une première et une deuxième extrémités ouvertes, et un flacon collecteur (11) destiné à recevoir la salive, et qui est relié de façon séparable à la première extrémité dudit tube, ledit flacon possédant une ouverture (14) à une première extrémité et un disque filtrant (16) qui couvre ladite ouverture, laquelle est en prise avec la première extrémité dudit tube (17), un élément piston (20) monté mobile dans la deuxième extrémité dudit tube cylindrique creux (17) de manière à comprimer une masse de ladite matière absorbante de la salive (9) reçue dans le tube cylindrique (17) entre ledit élément piston (20) et la première extrémité dudit tube cylindrique (17) qui est recouverte par le disque filtrant (16) lorsqu'on le déplace vers ledit disque filtrant (16) pour exprimer de cette façon la salive contenue dans ladite masse, de telle manière que la salive exprimée s'écoule dans ledit flacon collecteur (11) à travers le disque filtrant (16).

2. Appareil selon la revendication 1, caractérisé en ce qu'un espace de stockage (21) est formé dans ledit élément piston pour pouvoir sélectivement stocker temporairement ladite masse de matière absorbante de la salive (9).

3. Appareil selon la revendication 2, caractérisé en ce qu'un élément formant couvercle amovible (23) est assemblé de façon séparable audit élément piston (20) pour fermer ledit espace de stockage (21).

4. Appareil selon l'une quelconque des revendications 1 à 3, caractérisé en ce que ladite masse (9) de matière absorbante de la salive comprend une éponge cellulosique.

5. Appareil selon une quelconque des revendications 1 à 4, caractérisé en ce que des premiers filets de vis (19) sont formés sur la surface interne dudit tube cylindrique creux (17) et que des deuxième filets de vis (20A) sont formés sur la surface externe dudit élément piston (20), lequel est inséré télescopiquement dans la deuxième extrémité dudit tube (17) de telle manière que lesdits deuxièmes filets de vis (20A) se vissent dans lesdits premiers filets de vis (19).

## Patentansprüche

1. Gerät zum Sammeln eines Speichelvolumens als Probe von einer Testperson, mit einem Behälter zur Aufnahme von speichelabsorbierendem Material und mit einer Einrichtung zum Ausdrücken des Speichels aus dem speichelabsorbierendem Material, um diesen im Behälter zu sammeln, dadurch gekennzeichnet, daß der Behälter eine hohle zylindrische Büchse (17) mit ersten und zweiten offenen Enden und eine Sammelphiole (11) für die Aufnahme von Speichel aufweist, die lösbar an das erste Ende der Büchse angeschlossen ist, wobei die Phiole eine Ausnehmung (14) an einem Ende und eine diese Ausnehmung überdeckende Filterscheibe (16) aufweist, die mit dem ersten Ende der Büchse (17) in Verbindung steht, wobei ein Kolbenelement (20) bewegbar in das zweite Ende der hohlen zylindrischen Büchse (17) eingesetzt ist, so daß es eine in der zylindrischen Büchse (17) eingesetzt ist, so daß es eine in der zylindrischen Büchse (17) befindliche Masse des speichelabsorbierenden Materials (9) zwischen dem Kolbenelement (20) und dem von der Filterscheibe überdeckten ersten Ende der zylindrischen Büchse 17 zusammendrückt, wenn es gegen die Filterscheibe (16) bewegt wird, um dadurch den Speichelgehalt der Masse derart auszudrücken, daß der ausgedrückte Speichel durch die Filterscheibe (16) in die Sammelphiole (11) fließt.

2. Gerät nach Anspruch 1, dadurch gekennzeichnet, daß ein Vorratsraum (21) im Kolbenelement gebildet ist, um wahlweise vorübergehend die Masse des speichelabsorbierenden Materials (9) aufzubewahren.

3. Vorrichtung nach Anspruch 2, dadurch gekennzeichnet, daß ein bewegbares Abdeckelement (23) zum Verschließen des Vorratsraumes (21) lösbar am Kolbenelement (20) angebracht ist.

4. Gerät nach einem der Ansprüche 1 bis 3, dadurch gekennzeichnet, daß die Masse (9) aus speichelabsorbierendem Material einen Zellulose-Schwamm aufweist.

5. Gerät nach einem der Ansprüche 1 bis 4, dadurch gekennzeichnet, daß ein erstes Schraubengewinde (19) auf der Innenfläche der hohlen zylindrischen Büchse (17) und daß ein zweites Schraubengewinde (20a) auf der Außenfläche des Kolbenelementes (20) geformt sind, welches teleskopartig in das zweite Ende der Büchse (17) derart eingesteckt ist, daß das zweite Schraubengewinde (20a) in das erste Schraubengewinde (19) eingreift.

FIG. 1

FIG. 2